# EUROPEAN PATENT APPLICATION

(11) **EP 4 349 253 A1**
(43) Date of publication of application: **10.04.2024**
(21) Application number: 22200172.9
(22) Date of filing: 07.10.2022
(51) Int. Cl.: A61B 5/11, A61B 5/00

(54) **WRITING INSTRUMENT**

(71) Applicant: BIC Violex Single Member S.A., 145 69 Anoixi (GR)
(72) Inventor: Chrysanthakopoulos, Nikolaos, 145 69 Anoixi (GR); Antonakis, Ion - Ioannis, 145 69 Anoixi (GR)
(74) Representative: Peterreins Schley

(57) **Abstract**

In a first aspect, the present disclosure relates to a computer-implemented method for monitoring a fingertip strength of a writing instrument's user, comprising:
- monitoring an amount of holding pressure of at least one fingertip applied on the writing instrument (10) during writing with the writing instrument (10);
- comparing the monitored amount of pressure to historical pressure data from the same user or a group of users; and
- providing an indication to the user in case that at least two writing sessions from the user comprise reduced pressure on the writing instrument (10), when compared to the historical pressure data.

## Description

### Technical Field

The present disclosure relates to a method for monitoring a fingertip strength of a writing instrument's user and a writing instrument for monitoring a fingertip strength of its user.

### Background

There are multiple studies from different countries that are relating hand grip strength as a sign for potential cognitive impairment. Recent research has shown that in a study of more than 13000 older participants in the USA, for every 5 kg of reduction in handgrip strength, there was an association with 10% greater risk for any cognitive impairment.

Similar results were also found in a separate study in Korea with a sample size of more than 1000, again an association was shown between the reduction in handgrip strength and dementia. However, the researchers also showed that low BMI also affects (increases) the risk of dementia.

In a similar study in China, the researchers accumulated data and knowledge on the relationship between noncognitive features and cognition in populations with differing cognitive status and revealed that the stronger handgrip strength was associated with better performances on cognitive function. Hence, it can be speculated that handgrip strength can help early recognition of Chinese Alzheimer's Disease (AD) patients.

Additionally, multiple scoping reviews on the subject concluded that changes in the performance of motor tasks i.e., grip strength has been associated with changes in cognition and potential risk of dementia. Therefore, they claim that handgrip strength is a simple, reliable, and inexpensive means of monitoring the progression of cognitive decline in individuals if tested and reviewed on a consistent basis e.g., bimonthly and should be investigated further as a biomarker for cognitive decline in AD and Mild Cognitive Impairment (MCI).

Alzheimer's Disease (AD) is a form of dementia, a neurodegenerative disease of the brain. The disease process is associated with amyloid plaques, neurofibrillary tangles, and loss of neuronal connections in the brain. Unfortunately, the cause of Alzheimer's disease is poorly understood. There are many environmental and genetic risk factors associated with it. The strongest genetic risk factor is from an allele of APOE gene. Other risk factors are history of head injury, clinical depression, and high blood pressure.

Mild Cognitive Impairment (MCI) is an early stage of memory loss or other cognitive ability loss (such as language or visual/spatial perception) in individuals who maintain the ability to independently perform most daily activities. Individuals with MCI may have a higher risk of developing dementia.

### Summary

In a first general aspect, the present disclosure relates to a computer-implemented method for monitoring a fingertip strength of a writing instrument's user, comprising:
monitoring an amount of holding pressure of at least one fingertip applied on the writing instrument during writing with the writing instrument;
comparing the monitored amount of pressure to historical pressure data from the same user or a group of users; and
providing an indication to the user in case that at least two writing sessions from the user comprise reduced pressure on the writing instrument, when compared to the historical pressure data.

In embodiments, the method may comprise storing monitored pressure data as historical pressure data.

In embodiments, the method may comprise alerting the user in case of holding pressure reduction by activating a tactile feedback generator of the writing instrument.

In embodiments, the indication may be provided when the monitored amount of pressure is equal to or smaller than an average amount of historical pressure data.

In embodiments, holding pressure of more than one fingertip applied on the writing instrument may be monitored.

In embodiments, the method may comprise generating a value that is related to the user's overall handgrip strength from the monitored amounts of pressure; and comparing the value to historical values from the same user or a group of users.

In embodiments, the method may comprise indicating to the user, to hold the writing instrument with the palm and to grip the writing instrument with highest strength; monitoring an amount of holding pressure during the grip.

In embodiments, the indication may be provided via a user interface of the writing instrument.

In embodiments, the indication may be provided via a wireless communication system to an external device having an interface.

In a second general aspect, the present disclosure relates to a writing instrument for monitoring a fingertip strength of its user, comprising:
one or more sensors configured to monitor an amount of holding pressure of at least one fingertip applied on the writing instrument during writing with the writing instrument;
a communication system configured to provide an indication in case that at least two writing sessions from the user comprised reduced pressure on the writing instrument;
a storage system configured to store monitored pressure data as historical pressure data; and
a computer system configured to execute the computer-implemented method for monitoring a fingertip strength as described above.

In embodiments, the writing instrument may comprise a tactile feedback generator configured to alert the user in case of holding pressure reduction.

In embodiments, the one or more sensors may be configured to convert mechanical pressure into an electrical signal and are connected to the computer system for providing the electrical signal.

In embodiments, the one or more sensors may be arranged such that each finger in any potential grip configuration is differentiated and the applied pressure is monitored.

In embodiments, a gap area may be provided between two sensors to avoid an accidental contact of a finger with two sensors.

In embodiments, the writing instrument may comprise a user interface configured to receive input from a user and/or to provide information to the user such as the indication; and/or a wireless communication system configured to provide the indication via an external device having an interface.

Particular examples of the first to second general aspects can be implemented so as to realize one or more of the following advantages.

First, a system is provided for enabling the user to be notified on the potential of developing a cognitive disorder e.g., AD, MCI, etc. and seek medical attention to confirm the findings. The writing instrument can be considered and used as a wellness device. The fingertip strength and the handgrip strength are a general indicator of upper body strength and overall strength, as well as of the potential of cognitive impairment.

Second, the present disclosure enables a writing instrument such as a personal smart pen to constantly monitor the user's fingertip strength while they are writing and relate it to their handgrip strength. As the user is taking notes or performing tasks that require writing, over time, the device may create a profile that can be used as reference.

Third, if the user's handgrip and hence fingertip strength is reduced for multiple writing sessions, the pen can notify them and indicate the need for medical attention to confirm or deny the potential development of a cognitive disorder. Such smart pen system can adapt to the user's current state and does not need any additional maintenance.

Fourth, an everyday tool such as a pen would prove to be ideal for providing a platform for detecting any variance in the user's grip strength. Thus, a smart pen is enabled to measure and record user finger grip strength while writing or holding a pen.

Studies have shown that males normally can generate about 46 kg of handgrip force, and females about 23 kg; with a grip strength of only 4 kg is required to perform 90 percent of Activities of Daily Living (ADL). As a result, the system that is responsible for measuring the handgrip of the user may have a dynamic range of at least 500 Newton and with a resolution of at least 10 Newton increments. There are multiple technologies that can be used to measure compressive force, ranging from piezoelectric materials, to strain gauges and load cells. In addition to the aforementioned sensors, optical fiber force sensors are based on optical interferometry, the phase modulation of light, and offer very high accuracy under a wide range of operating conditions and number of cycles (compression cycles).

Regarding pressure or force sensors, piezoelectric (PE) crystals (e.g. Cellulose microfiber or flexible liquid metal-tin sulfide) may be employed, that can deliver high electrical throughput and power density under repeated mechanical stress as for example in the case of hand punching for portable items to power low-power electronics. These PE nanogenerators can generate voltage in the range of milli-Volts (50-120mV) with a power output of several micro-Watts. Said materials can be used for measuring the force applied by the user, as their voltage output is related to the pressure applied.

With the rise in sales of portable devices and more especially wearables, and the consumer need for thinner and smaller devices, haptic and tactile feedback technologies have been miniaturized to the degree that they can fit in a small form-factor device. Such devices range from vibration motors to linear actuators and piezoelectric crystals, which can accomplish complex haptic sensations with a low-power requirements.

Regarding writing action, handwriting is the writing action done with a writing instrument, such as a pen or pencil, using the hand. Handwriting includes both printing and cursive styles. Handwriting involves the use of a pen or pencil which the user keeps holding with his hand/fingers even when he does not write.

The normal grip of a writing instrument may be performed in the following way:
- the writing instrument which may be a pen or a pencil is held in a stable position between the thumb, index and middle fingers,
- the ring and little fingers are bent and rest comfortably on the table,
- the index finger and thumb form an open space,
- the wrist is bent back slightly, and the forearm is resting on the table,
- the writing instrument is held about 1- 2 cm from the tip.

So, especially while writing, a user exerts a gripping force on the body of the pen.

Certain terms are used in the following manner in the present disclosure:
The expression "digital device" may refer to an electronic device that uses discrete, numerable data and processes for all its operations, and is capable of displaying content to the user. Examples of such a device include but are not limited to: Mobile phones, Laptops, Tablets, Personal computers, Netbooks, iPads, etc.

The expression "haptic or tactile feedback" may refer to a physical response on a digital device from the user input, also known as kinaesthetic communication. The application with mechanical or electronic means of force, vibrations, or motions to the user in response to a user's action on the digital device.

The expression "human machine interface" may refer to a user interface or dashboard or an input system that connects a person to a machine, system, or device. For example, a physical button on a remote controller.

The expression "group of users" may refer to a panel of users selected through specific methodology, with the scope to test products and provide feedback, in order to provide statistical insights to specific parameters of the products and their respective use.

### Description of the Drawings

**Fig. 1** illustrates an overview of components of a writing instrument according to the present disclosure.
**Fig. 2** illustrates a process flow diagram of a writing instrument according to the present disclosure.
**Fig. 3** illustrates a perspective view of a writing instrument according to the present disclosure.
**Fig. 4** illustrates a perspective view of a writing instrument according to the present disclosure.
**Fig. 5** illustrates a method flow chart for monitoring a fingertip strength of a writing instrument's user according to the present disclosure.

### Detailed Description

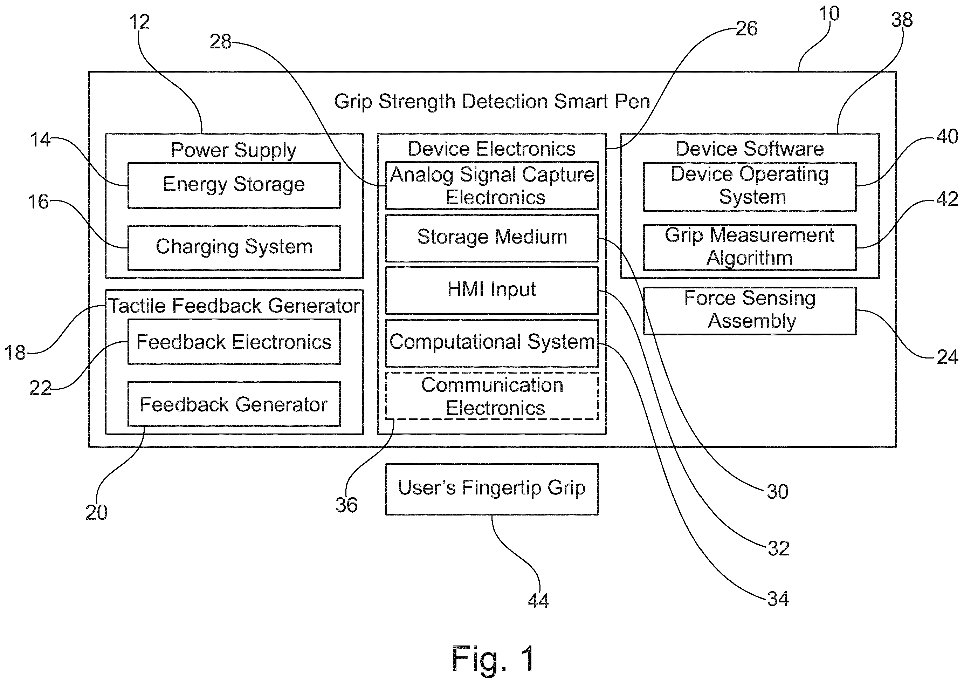

**Fig. 1** shows an overview of components of a writing instrument 10 according to the present disclosure. The writing instrument 10 may be named a grip strength detection smart pen, digital writing instrument or digital device.

An example of the writing instrument 10 enables the user to monitor their fingertip grip strength while they are using the writing instrument 10 and alert them in case their strength has reduced, which could indicate the potential of a cognitive disorder.

As shown in **Fig. 1****,** the writing instrument 10 includes a power supply 12 to provide the energy for the following sub-systems of the writing instrument 10. The power supply 12 includes a subsystem energy storage 14 which is the energy source of the writing instrument 10 and can be of the form of a battery.

The power supply 12 includes a subsystem charging system 16 which includes the electronics for charging and maintaining the health of the energy storage 14.

The writing instrument 10 may include a subsystem tactile feedback generator 18 that is capable of receiving digital data and converting them into vibrations that can be felt by the user. Hence, its location can be as close to the user's finger tips as possible i.e., close to the tip of a digital pen.

The tactile feedback generator 18 may include a subsystem feedback generator 20 that is responsible for converting the analog electrical signals i.e., electrical energy into mechanical energy in the form of vibrations that can be felt by the user i.e., the user's fingertips. The subsystem may generate vibrations in the range of 0 to 500 Hz with a controllable amplitude, while being power efficient enough to be integrated within a portable consumer product.

The feedback generator 20 can be of the form but not limited to at least one of rotary motors with a weight off the center of rotation, linear vibration motors, and piezoelectric vibration motors.

The tactile feedback generator 18 may include feedback electronics 22 that include the hardware i.e., motor drivers and firmware for converting the digital signal e.g., in the form of binary into an electrical e.g., analog signal that can drive the feedback generator 20.

The writing instrument 10 may include a subassembly force sensing assembly 24 that encompasses the hardware required for converting mechanical pressure into an electrical signal.

In examples of the present disclosure, this subassembly 24 is located at the grip of the writing instrument 10 i.e., the location where the user usually rests and grips the pen with his fingers during their writing. The force sensing assembly 24 may be segmented such that each finger in any potential grip 44 can be differentiated and the force applied can be captured. For example, when a user is holding the pen in a "dynamic tripod" configuration, (where the user rests their pen on their middle finger and hold their thumb on the pen to steady their handwriting), there are 3 points of contact.

Therefore, the force sensing assembly 24 may be segmented in at least 3 segments, such that each finger's pressure is accurately measured. It should be noted that by increasing the segments that can measure the applied force, the system will be capable of differentiating between different grip styles/configurations and hence improve the overall granularity of the measurements.

The type of the force sensors could be of the form but not limited to at least one of:
i. piezoelectric materials
ii. strain gauges
iii. load cells
iv. optical fiber force sensors
v. piezoelectric (PE) nanogenerators
vi. Force sensing resistors
vii. Ultrasonic force sensors

In examples of the present disclosure, the width of each sensor may range from 1 mm to 10 mm.

In examples of the present disclosure, the length of each sensor may range from 0,5 cm to 12 cm.

In examples of the present disclosure, the sensors may be flat or may be curved to accommodate the shape of the writing instrument 10.

In examples of the present disclosure, the sensors may be adjacent to each other or optionally a gap may exist between them to avoid an accidental contact of a finger with two sensors.

In examples of the present disclosure, the gap may be made of plastic or rubber material.

In examples of the present disclosure, the width of the gap may range from 1 mm to 10 mm.

In examples of the present disclosure, the gap surface may be at the same level with the sensors surface, or more preferably may be more elevated or may form a recess relative to the sensors surface.

In examples of the present disclosure, the height of the elevation and/or recess of the gap may range from 1 mm to 10 mm.

In examples of the present disclosure, the surface of the gap may be flat or may be curved or may be pointy in order to make the user limit his finger position in one of the sensor areas of the writing instrument 10.

The writing instrument 10 may include a component writing instrument electronics or device electronics 26 which enables the writing instrument 10 to function.

The device electronics 26 may include a subcomponent analog signal capture electronics 28 that encompasses all electronics including the physical and digital signal filters and the analog to digital converter A2D as well as firmware to capture an analog signal from the force sensing assembly 24 and convert it into a digital format.

The device electronics 26 may include a subcomponent storage medium 30 that includes the hardware, firmware and software for the writing instrument 10 to store data captured by the system and more specifically the analog signal capture electronics 28. Moreover, this subcomponent allows the writing instrument operating system or device operating system 40 to load said data and utilize it for a wide range of uses e.g., data comparison, data viewing, data deletion, etc.

The storage medium 30 could be of the form but not limited to at least one of magnetic storage, optical storage, and solid state storage (e.g., NAND Flash, NOR Flash, etc.).

The device electronics 26 may include a subsystem human machine interface (HMI) input 32 that includes all of the hardware and software for the writing instrument 10 to accept human input and display its state to the user. The physical input interface of the writing instrument 10 could be of the form of a button, a rotational encoder wheel, or a linear slider. The display element of this interface can be at least one LED light of single or multiple colors. The input and display interfaces are read and controlled by the device operating system 40.

The device electronics 26 may include a subsystem computational system 34 that includes the hardware for the writing instrument 10 to function. The main function of this subsystem is to run the device operating system 40 and coordinate the hardware of the writing instrument 10 such that the user's requests and inputs to the writing instrument 10 are recognized and executed accordingly. In addition, it enables the writing instrument's 10 functionality via executing the writing instrument's 10 main software regarding the storage medium 30 and the human machine interface (HMI) input 32. Said subsystem include the Central Processing Unit (CPU), as well as the Random-Access Memory (RAM) and Read Only Memory (ROM).

The device electronics 26 may include a subcomponent communication electronics 36 for establishing a wireless connection between the digital writing instrument 10 and other devices such as a digital device, a mobile phone, a personal computer, etc.). The communication protocol could be of the form but not limited to at least one of Bluetooth, NFC, and Wireless Fidelity (WiFi).

The communication electronics 36 may include or may be in communication with an HMI display component of the writing instrument 10. It should be noted that the HMI display component can be used for displaying relevant information to the user such as at least one of the writing instrument's 10 status (ON or OFF), the selected vibration pattern's intensity (e.g., blue color = low amplitude, red = high/maximum amplitude), etc.

Building upon the notification system of the feedback electronics 22, it is possible to utilize the optional communication electronics 36 on the smart pen device and notify the user of their reduction in hand grip strength via an application on their mobile phone. Additionally, this system may enable the user to visually see a graph of their measurements over time or even the raw captured data.

The writing instrument 10 may further include a subcomponent digital software 38.

The subcomponent digital software 38 includes a software component device operating system 40 also known as Operating System (OS) that manages the writing instrument's 10 hardware, software resources, and provides common services for application specific algorithms.

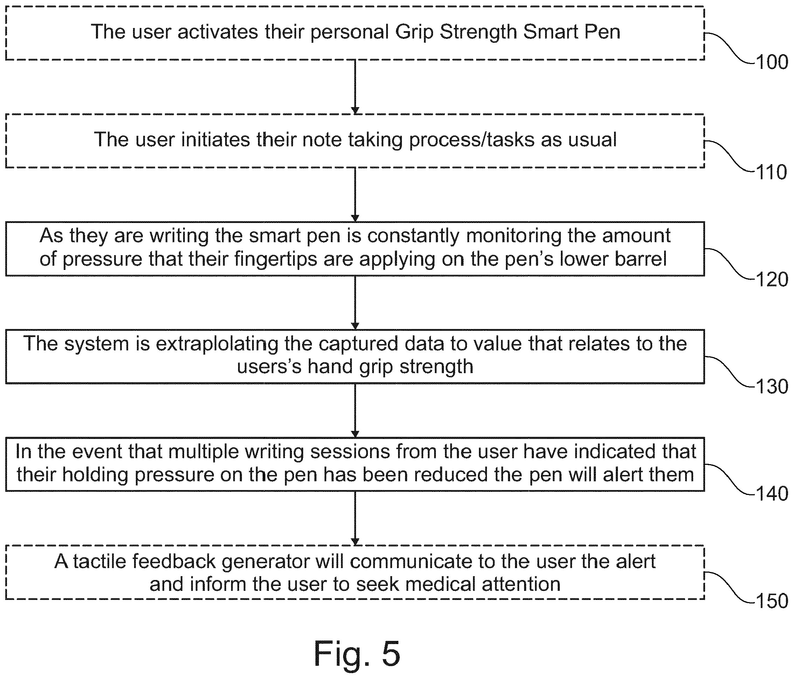

The subcomponent digital software 38 may include an algorithm grip measurement algorithm 42 that is responsible for utilizing the data from the force sensing assembly 24 and generating a value that is related to the user's overall handgrip strength. Said algorithm is running in the background constantly and comparing its currently generated value to user specific or general population historical data that are read from the storage medium 30. Said algorithm may include or consist of the method for monitoring a fingertip strength of a writing instrument's user an example of which is depicted in Fig. 5.

The value generated in the last sessions e.g., at least two consequent writing sessions or more can be significantly lower than the average value of the historical data e.g., more than the equivalent of 4 kg hand grip pressure. In such example, if the user usually grips the pen with 10 N, the pen could relate this to an overall hand grip strength of 25 kg. If at another timepoint, the pen measures 8 N on its grip, this could be related to 20 kg of overall hand grip strength. Then, a signal is sent to the tactile feedback generator 18 or a mobile device in order to inform the user of the potential of a cognitive disorder. It should be noted that during the first sessions, the smart pen may be creating a baseline in a calibration process, and it might not be capable of providing feedback to the user.

The user's fingertip grip 44 depicts the physical force applied by the user due to the contact of the user's finger tips with a main body or a grip section of the writing instrument 10.

To improve the accuracy of the entire system, it is possible to extend the force sensing array on the writing instrument 10 to cover the entire extent of the pen, i.e., from the nib to the top of the pen. The sensors can be of the same type as the ones described. However, the method could be adjusted, by adding an addition step where the user will be required to hold the writing instrument with their entire palm and grip it as hard as possible. This will provide further data to the system, and it will allow it to calibrate its results.

Additionally, or optionally, in the event that the pen identifies a reduction in hand grip strength from the way that the user has been holding the pen while writing, it could ask the user to proceed with an additional test of holding the pen with their entire palm and apply as much force as possible. Assuming that the pen has historical data from the user's hand grip, it can confirm or deny the current reduction of pressure (and hence the potential of cognitive disorder).

According to examples of the present disclosure, a user may be enabled to be notified over the potential of developing a cognitive disorder via the use of a smart writing instrument or a pen with grip strength sensors. Such system or writing instrument monitors and tracks the user's grip strength over time. An array of force sensors on specific pen body areas that come in constant contact with the user's fingertips measure the applied force over time and over specific tasks and compare it with user's historical data. If the force data captured deviate significantly, the user is alerted.

The user may initiate their note taking process as usual. As they write down their notes or everyday tasks, the pen monitors the force that they apply with their fingertips on the lower pen barrel (writing instrument grip area). This is achieved by an array of force sensors covering the entire area that the user's fingertips can come in contact with the pen. Said sensors are capable of capturing the force data with a fairly high accuracy over a wide range in order to ensure that any variances can be identified. The signals from the sensors are transferred to and analyzed by the on-board electronics.

The user may be alerted by a mobile device connected to the smart pen or alternatively by a tactile feedback mechanism on the pen. The tactile feedback mechanism is capable of generating frequencies below 500Hz and is located as close to the tip of the writing instrument as possible, to ensure that the vibrations are felt by the user's fingertips as fingertips are sensitive enough that can differentiate and identify a wide range of vibration frequencies.

The device may also incorporate the required electronics and software drivers for connecting wirelessly (i.e., Bluetooth, Wi-Fi, etc.) to an optional supplementary device.

The system may now in real time, capture the griping strength of the user while they are holding the pen which can be related to the entire handgrip strength and compare that data to previous known historical data on that specific user's average grip strength. If the current grip pressure deviates significantly from the historical average, for multiple sessions, the writing instrument will notify the user by a mobile device app alert or via a series of tactile vibrations on the pen. This alert will signal the user to go and seek medical attention.

Hence, examples of the present disclosure may provide at least one of the following features: A digital writing instrument with sensors that can capture the pressure/force applied by the user's fingertips and relate it to the user's hand grip strength; a storage medium and system for storing the historical grip pressure data over the devices lifetime; an algorithm responsible for comparing the current grip pressure data to the historical ones and hence providing an indication to the user in the event that their estimated grip strength has been reduced (which could be associated with dementia or cognitive disorders); and a tactile feedback generator on the writing instrument that can alert the user of significant grip force reduction.

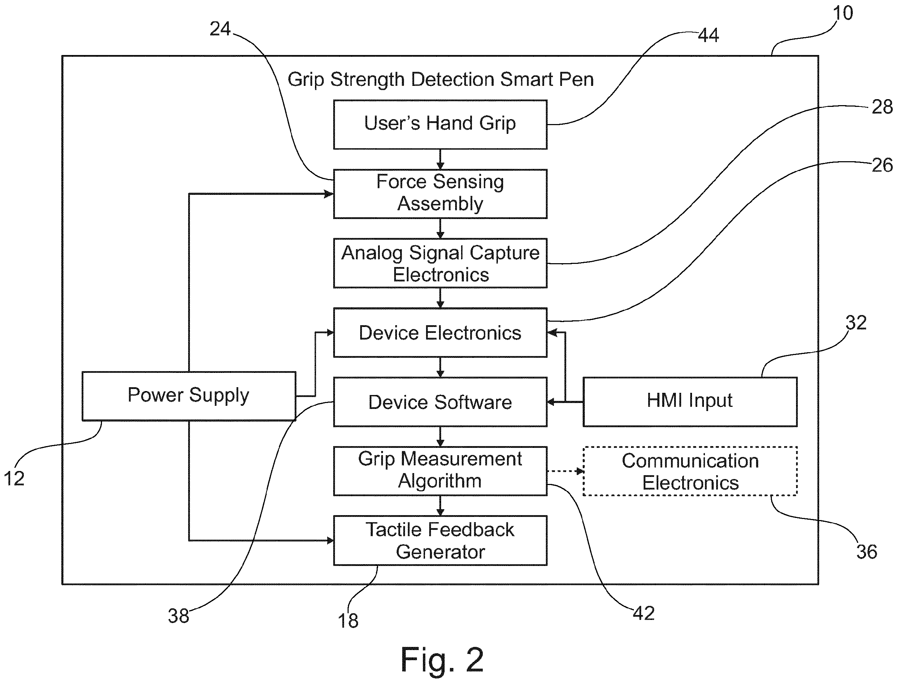

**Fig. 2** illustrates a process flow diagram of a writing instrument 10 according to the present disclosure.

The power supply 12 of the writing instrument 10 supplies power to the force sensing assembly 24, the device electronics 26, and the tactile feedback generator 18. The HMI input 32 supplies the device electronics 26 and the device software 38 with user input. Once a user's handgrip 44 is detected at the writing instrument 10 the force sensing assembly 24 detects the force and/or the pressure of the user's handgrip. The initial handgrip detection is executed by the force sensing assembly 24.

Values or parameters generated by the force sensing assembly 24 are received and processed by the analog signal capture electronics 28. In case of digital signals from the force sensing assembly 24 the analog signal capturing can be skipped. The digital signals are fed into the device electronics 26 and passed to the device software 38. Then, the digital values are input to the grip measurement algorithm 42.

The grip measurement algorithm 42 compares the measured pressure data to historical pressure data from the same user or group of users and decides whether the measured data deviates significantly from the historical data. If the measured data deviates significantly from the historical data by e.g., 20% the tactile feedback generator 18 is started to provide an indication to the user. Alternatively, or additionally, the outcome of the comparison is supplied to the communication electronics 36 which in turn provide the outcome of the comparison to digital device of the user.

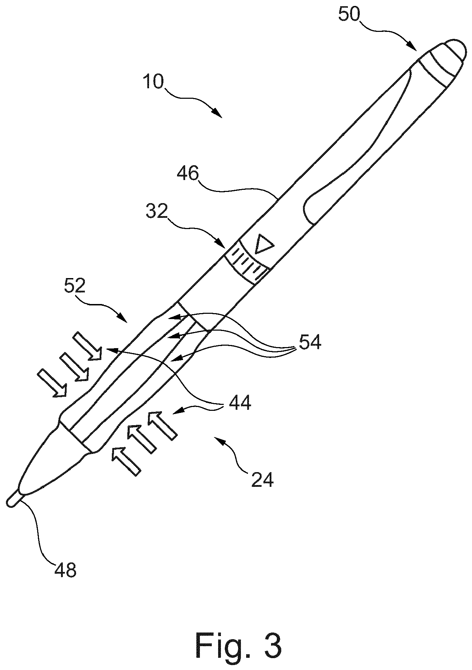

**Fig. 3** illustrates a perspective view of a writing instrument 10 according to the present disclosure. The writing instrument 10 can correspond to the writing instrument 10 as depicted in **Fig. 1** or **Fig. 2****.**

The writing instrument 10 includes a barrel shaped body 46 with a writing tip 48.the writing tip 48 could be an ink writing tip or a digital writing tip. The HMI input 32 includes in this example as selection wheel as well as the marker e.g. in form of a pointer orientated towards the selection wheel. The writing instrument 10 further includes an HMI display or HMI output 50 which is capable of indicating information like e.g., status information of the writing instrument 10 to the user. In this example, the HMI output 50 is arranged at an end opposite to the writing tip 48 and has the form of an LED strip.

The writing instrument 10 further includes a grip section 52 which is located close to the writing tip 48. The grip section 52 includes or consists of the force sensing assembly 24. The force sensing assembly 24 includes a force sensing array of one or more sensors 54 to monitor an amount of holding pressure of holding force of the fingertip of fingertips applied on the writing instrument 10 during a writing session.

In this specific example, three force sensors 54 are placed in the grip section. Thus, the force sensing array is segmented such that different grip types can be recognized and the respective fingertip forces can be measured.

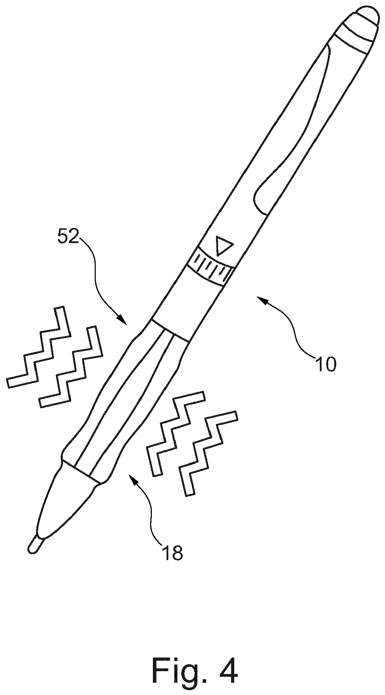

**Fig. 4** illustrates a perspective view of a writing instrument 10 according to the present disclosure. **Fig. 4** could depict different aspects of the writing instrument 10 shown in **Fig. 3****.**

The writing instrument 10 includes a tactile feedback generator 18 which is arranged in the grip section 52. The tactile feedback generator 18 may be arranged underneath or inside the force sensing assembly 24. The tactile feedback generator 18 may be part of a communication system or is a communication system to provide an indication in case that at least two writing sessions from the user comprised reduced pressure on the writing instrument. Thus, the tactile feedback generator 18 can alert the user in case of holding pressure reduction.

**Fig. 5** illustrates a method flow chart for monitoring a fingertip strength of a writing instrument's user according to the present disclosure.

In examples, during a step 100 of the method, the user activates their personal writing instrument 10. Such activation may be achieved by a user input e.g., via the HMI input 32. It is also possible that the writing instrument 10 automatically detects activation by the force sensing assembly 24.

In examples, during a step 110, the user initiates their note taking process or tasks as usual.

In examples, during a 120, as they are writing the writing instrument 10 is constantly monitoring the amount of pressure that their fingertips are applying on the writing instrument's 10 lower barrel especially the grip section 52. An array of force sensors 54 is capturing accurate data from each finger or fingertip. In other words, the third step 120 includes monitoring an amount of holding pressure of at least one fingertip applied on the writing instrument 10 during writing with the writing instrument 10.

In examples, during a step 130, the captured data is extrapolated to a value that relates to the user's hand grip strength. In the background the writing instrument 10 is comparing the newly captured and extrapolated data to historical ones from the same user or a group of relevant users.

In other words, the third step 130 includes comparing the monitored amount of pressure to historical pressure data from the same user or a group of users.

In examples, during a step 140, in the event that multiple writing sessions from the user have indicated that their holding pressure on the writing instrument 10 has been reduced the writing instrument 10 will alert them. Reduction in grip strength is associated with increased risk of dementia or cognitive disorders. In other words, the third step 140 includes providing an indication to the user in case that at least two writing sessions from the user comprise reduced pressure on the writing instrument 10, when compared to the historical pressure data.

In examples, during a step 150, a tactile feedback generator communicates to the user the alert or the indication and may inform the user to seek medical attention. The user can optionally adjust the amplitude of the vibrations of the feedback generator via the HMI input 32.

The present disclosure also relates to the computer-implemented method for monitoring a fingertip strength of a writing instrument's user and the writing instrument for monitoring a fingertip strength of its user of the following aspects:
1. A computer-implemented method for monitoring a fingertip strength of a writing instrument's user, comprising:
   - monitoring an amount of holding pressure of at least one fingertip applied on the writing instrument during writing with the writing instrument;
   - comparing the monitored amount of pressure to historical pressure data from the same user or a group of users; and
   - providing an indication to the user in case that at least two writing sessions from the user comprise reduced pressure on the writing instrument, when compared to the historical pressure data.
2. The method of aspect 1, comprising:
   - storing monitored pressure data as historical pressure data.
3. The method of one of the preceding aspects, wherein the indication is provided in case that at least the two most recent writing sessions from the user comprised reduced pressure on the writing instrument.
4. The method of one of the preceding aspects, comprising:
   - alerting the user in case of holding pressure reduction by activating a tactile feedback generator of the writing instrument.
5. The method of one of the preceding aspects, wherein the indication is provided when the monitored amount of pressure is equal to or smaller than 80 % of an average amount of historical pressure data.
6. The method of one of the preceding aspects, wherein holding pressure of more than one fingertip applied on the writing instrument is monitored.
7. The method of aspect 6, comprising:
   - generating a value that is related to the user's overall handgrip strength from the monitored amounts of pressure; and
   - comparing the value to historical values from the same user or a group of users.
8. The method of one of the preceding aspects, comprising:
   - indicating to the user, to hold the writing instrument with the palm and to grip the writing instrument with highest strength;
   - monitoring an amount of holding pressure during the grip.
9. The method of one of the preceding aspects, wherein the indication is provided via a user interface of the writing instrument.
10. The method of one of the preceding aspects, wherein the indication is provided via a wireless communication system to an external device having an interface.
11. A writing instrument for monitoring a fingertip strength of its user, comprising:
   - one or more sensors configured to monitor an amount of holding pressure of at least one fingertip applied on the writing instrument during writing with the writing instrument;
   - a communication system configured to provide an indication in case that at least two writing sessions from the user comprised reduced pressure on the writing instrument;
   - a storage system configured to store monitored pressure data as historical pressure data and
   - a computer system configured to execute the computer-implemented method for monitoring a fingertip strength according to one of the preceding aspects.
12. The writing instrument of aspect 11, comprising:
   - a tactile feedback generator configured to alert the user in case of holding pressure reduction.
13. The writing instrument of aspect 12, wherein the tactile feedback generator is configured to generate vibrations in the range of 0 to 500Hz with a controllable amplitude.
14. The writing instrument of one of aspects 11 to 13, wherein the one or more sensors are configured to convert mechanical pressure into an electrical signal and are connected to the computer system for providing the electrical signal.
15. The writing instrument of one of aspects 11 to 14, wherein the one or more sensors are located in a grip area of the writing instrument.
16. The writing instrument of one of aspects 11 to 15, wherein the one or more sensors are arranged such that each finger in any potential grip configuration is differentiated and the applied pressure is monitored.
17. The writing instrument of one of aspects 11 to 16, wherein a gap area is provided between two sensors to avoid an accidental contact of a finger with two sensors.
18. The writing instrument of aspect 17, wherein a surface of the gap area is elevated or receded relative to a surface of the sensors.
19. The writing instrument of one of aspects 11 to 18, comprising:
   - a user interface configured to receive input from a user and/or to provide information to the user such as the indication; and/or
   - a wireless communication system configured to provide the indication via an external device having an interface.

## Claims

1. A computer-implemented method for monitoring a fingertip strength of a writing instrument's user, comprising:
- monitoring an amount of holding pressure of at least one fingertip applied on the writing instrument (10) during writing with the writing instrument (10);
- comparing the monitored amount of pressure to historical pressure data from the same user or a group of users; and
- providing an indication to the user in case that at least two writing sessions from the user comprise reduced pressure on the writing instrument (10), when compared to the historical pressure data.

2. The method of claim 1, comprising:
- storing monitored pressure data as historical pressure data.

3. The method of one of the preceding claims, comprising:
- alerting the user in case of holding pressure reduction by activating a tactile feedback generator (18) of the writing instrument (10).

4. The method of one of the preceding claims, wherein the indication is provided when the monitored amount of pressure is equal to or smaller than an average amount of historical pressure data.

5. The method of one of the preceding claims, wherein holding pressure of more than one fingertip applied on the writing instrument (10) is monitored.

6. The method of claim 5, comprising:
- generating a value that is related to the user's overall handgrip (44) strength from the monitored amounts of pressure; and
- comparing the value to historical values from the same user or a group of users.

7. The method of one of the preceding claims, comprising:
- indicating to the user, to hold the writing instrument (10) with the palm and to grip (44) the writing instrument (10) with highest strength;
- monitoring an amount of holding pressure during the grip (44).

8. The method of one of the preceding claims, wherein the indication is provided via a user interface of the writing instrument (10).

9. The method of one of the preceding claims, wherein the indication is provided via a wireless communication system (36) to an external device having an interface.

10. A writing instrument (10) for monitoring a fingertip strength of its user, comprising:
- one or more sensors (54) configured to monitor an amount of holding pressure of at least one fingertip applied on the writing instrument (10) during writing with the writing instrument (10);
- a communication system (18, 36) configured to provide an indication in case that at least two writing sessions from the user comprised reduced pressure on the writing instrument (10);
- a storage system (30) configured to store monitored pressure data as historical pressure data; and
- a computer system (26, 38) configured to execute the computer-implemented method for monitoring a fingertip strength according to one of the preceding claims.

11. The writing instrument (10) of claim 10, comprising:
- a tactile feedback generator (18) configured to alert the user in case of holding pressure reduction.

12. The writing instrument (10) of claim 10 or 11, wherein the one or more sensors (54) are configured to convert mechanical pressure into an electrical signal and are connected to the computer system (26, 38) for providing the electrical signal.

13. The writing instrument (10) of one of claims 10 to 12, wherein the one or more sensors (54) are arranged such that each finger in any potential grip (44) configuration is differentiated and the applied pressure is monitored.

14. The writing instrument (10) of one of claims 10 to 13, wherein a gap area is provided between two sensors (54) to avoid an accidental contact of a finger with two sensors (54).

15. The writing instrument (10) of one of claims 10 to 14, comprising:
- a user interface (32, 50) configured to receive input from a user and/or to provide information to the user such as the indication; and/or
- a wireless communication system (36) configured to provide the indication via an external device having an interface.
